# DEMANDE DE BREVET EUROPEEN

(11) **EP 3 207 918 A1**
(43) Date de publication de la demande: **23.08.2017**
(21) Numéro de dépôt: 17158150.7
(22) Date de dépôt: 16.12.2010
(51) Int. Cl.: A61K 8/31, A61K 8/86, A61K 8/87, A61Q 5/12, A61K 8/81

(54) **UTILISATION D'UNE COMPOSITION COSMÉTIQUE CONTENANT UN ALCANE LINÉAIRE VOLATIL ET UN POLYMÈRE ASSOCIATIF NON IONIQUE POUR LE CONDITIONNEMENT DES CHEVEUX**

(30) Priorité: 23.12.2009 FR 0959481
(62) Demande divisionnaire de: 10195381.8
(71) Demandeur: L'OREAL, 75008 Paris (FR)
(72) Inventeur: DESENNE, Patricia, 74370 PRINGY (FR); BOURDIN, Claire, 92300 LEVALLOIS PERRET (FR)
(74) Mandataire: Casalonga

(57) **Abrégé**

L'invention concerne l'utilisation pour le conditionnement des fibres kératiniques, en particulier des cheveux, d'une composition cosmétique comprenant dans un milieu cosmétiquement acceptable un ou plusieurs alcanes linéaires volatils et un ou plusieurs polymères associatifs non ioniques.

## Description

La présente invention se rapporte à l'utilisation d'une composition cosmétique comprenant dans un milieu cosmétiquement acceptable un ou plusieurs alcanes linéaires volatils et un ou plusieurs polymères associatifs non-ioniques pour le conditionnement des fibres kératiniques, en particulier des cheveux.

De nombreux produits de soin capillaire ont été décrits dans l'art antérieur. Les compositions correspondantes ont pour but d'apporter de bonnes propriétés cosmétiques aux cheveux.

Il est connu d'utiliser des solvants volatils dans les produits capillaires de soin rincés ou non rincés. Ces solvants volatils permettent généralement de modifier le rendu sensoriel d'un produit capillaire en lui conférant une structure légère et non collante dans la main. Ils lui confèrent également un caractère glissant qui facilite sa répartition sur les cheveux, et en particulier sur les cheveux secs.

De plus, dans les compositions sous forme d'émulsion aqueuse de type huile-dans-eau, qui se présentent sous la forme de crèmes plus ou moins gélifiées, l'ajout de solvants volatils peut également permettre de solubiliser des gommes de silicone qui, sans cela, sont difficiles à introduire dans les compositions à cause de leur viscosité intrinsèque.

Toutefois, de tels solvants volatils, en particulier les esters gras liquides, les huiles hydrocarbonées de type isododécane ou isohexadécane et les huiles siliconées de type cyclométhicone, peuvent notamment induire des problèmes de toucher gras, de manque de brillance, de cheveux raidis, durs.

On connaît par ailleurs des documents DE 10 2008 017 031 et WO 2007/068371 des compositions de protection solaire comprenant un mélange d'alcanes linéaires volatils et un polymère associatif non ionique du type copolymère de vinylpyrrolidone.

Ainsi, il existe un besoin de fournir des compositions cosmétiques de soin capillaire qui permettent d'améliorer le lissage et la brillance sur cheveux humides, qui transforment la fibre kératinique lors du rinçage éventuel, qui facilitent le passage d'une brosse lors d'un brossage, et qui permettent d'améliorer la douceur, le lissage, la brillance, la souplesse et la légèreté sur cheveux secs.

La demanderesse a découvert de façon surprenante que l'association d'un ou plusieurs alcanes linéaires volatils et d'un ou plusieurs polymères associatifs non-ioniques pouvait être utilisée pour résoudre les problèmes de l'art antérieur et conduisait aux effets recherchés mentionnés précédemment.

Ainsi, l'invention a pour objet l'utilisation pour le conditionnement des fibres kératiniques, en particulier des cheveux, d'une composition cosmétique comprenant dans un milieu cosmétiquement acceptable un ou plusieurs alcanes linéaires volatils et un ou plusieurs polymères associatifs non-ioniques.

La composition utilisée selon l'invention contient un ou plusieurs alcane(s) linéaire(s) volatil(s). Par « un ou plusieurs alcane(s) linéaire(s) volatil(s) », on entend indifféremment « une ou plusieurs huile alcane(s) linéaire(s) volatile(s) ».

Un alcane linéaire volatil convenant à l'invention est liquide à température ambiante (environ 25 °C) et à la pression atmosphérique (760 mm Hg).

Par « alcane linéaire volatil » convenant à l'invention, on entend un alcane linéaire, susceptible de s'évaporer au contact de la peau en moins d'une heure, à température ambiante (25°C) et pression atmosphérique (760 mm Hg, c'est-à-dire 101 325 Pa), liquide à température ambiante, ayant notamment une vitesse d'évaporation allant de 0,01 à 15 mg/cm²/min, à température ambiante (25°C) et pression atmosphérique (760 mm Hg).

De préférence, le ou les alcanes linéaires volatils convenant à l'invention présentent une vitesse d'évaporation allant de 0,01 à 3,5 mg/cm²/min, à température ambiante (25°C) et pression atmosphérique (760 mm Hg).

De façon préférée, le ou les alcanes linéaires volatils convenant à l'invention présentent une vitesse d'évaporation allant de 0,01 à 1,5 mg/cm²/min, à température ambiante (25°C) et pression atmosphérique (760 mm Hg).

De façon plus préférée, le ou les alcanes linéaires volatils convenant à l'invention présentent une vitesse d'évaporation allant de 0,01 à 0,8 mg/cm²/min, à température ambiante (25°C) et pression atmosphérique (760 mm Hg).

De façon encore préférée, le ou les alcanes linéaires volatils convenant à l'invention présentent une vitesse d'évaporation allant de 0,01 à 0,3 mg/cm²/min, à température ambiante (25°C) et pression atmosphérique (760 mm Hg).

De façon encore préférée, le ou les alcanes linéaires volatils convenant à l'invention présentent une vitesse d'évaporation allant de 0,01 à 0,12 mg/cm²/min, à température ambiante (25°C) et pression atmosphérique (760 mm Hg).

La vitesse d'évaporation d'un alcane volatil conforme à l'invention (et plus généralement d'un solvant volatil) peut être notamment évaluée au moyen du protocole décrit dans WO 06/013413, et plus particulièrement au moyen du protocole décrit ci-après.

On introduit dans un cristallisoir (diamètre : 7 cm) placé sur une balance se trouvant dans une enceinte d'environ 0,3 m³ régulée en température (25 °C) et en hygrométrie (humidité relative 50 %) 15 g de solvant hydrocarboné volatil.

On laisse le liquide s'évaporer librement, sans l'agiter, en assurant une ventilation par un ventilateur (PAPST-MOTOREN, référence 8550 N, tournant à 2700 tours/minute) disposé en position verticale au-dessus du cristallisoir contenant le solvant hydrocarboné volatil, les pales étant dirigées vers le cristallisoir, à une distance de 20 cm par rapport au fond du cristallisoir.

On mesure à intervalles de temps réguliers la masse de solvant hydrocarboné volatil restante dans le cristallisoir.

On obtient alors le profil d'évaporation du solvant en traçant la courbe de la quantité de produit évaporé (en mg/cm²) en fonction du temps (en min).

Puis on calcule la vitesse d'évaporation qui correspond à la tangente à l'origine de la courbe obtenue. Les vitesses d'évaporation sont exprimées en mg de solvant volatil évaporé par unité de surface (cm²) et par unité de temps (minute).

Selon un mode de réalisation préféré, le ou les alcanes linéaires volatils convenant à l'invention ont une pression de vapeur (appelée également pression de vapeur saturante) non nulle, à température ambiante, en particulier une pression de vapeur allant de 0,3 Pa à 6000 Pa.

De façon préférée, le ou les alcanes linéaires volatils convenant à l'invention ont une pression de vapeur allant de 0,3 à 2000 Pa, à température ambiante (25°C).

De façon préférée, le ou les alcanes linéaires volatils convenant à l'invention ont une pression de vapeur allant de 0,3 à 1000 Pa, à température ambiante (25°C).

De façon plus préférée, le ou les alcanes linéaires volatils convenant à l'invention ont une pression de vapeur allant de 0,4 à 600 Pa, à température ambiante (25°C).

De façon préférée, le ou les alcanes linéaires volatils convenant à l'invention ont une pression de vapeur allant de 1 à 200 Pa, à température ambiante (25°C).

De façon encore préférée, le ou les alcanes linéaires volatils convenant à l'invention ont une pression de vapeur allant de 3 à 60 Pa, à température ambiante (25°C).

Selon un mode de réalisation, le ou les alcanes linéaires volatils convenant à l'invention peuvent présenter un point éclair compris dans l'intervalle variant de 30 à 120 °C, et plus particulièrement de 40 à 100 °C. Le point éclair est en particulier mesuré selon la Norme iso 3679.

Selon un mode de réalisation, le ou les alcanes linéaires volatils convenant à l'invention peuvent être les alcanes linéaires comprenant de 7 à 15 atomes de carbone.

De façon préférée, le ou les alcanes linéaires volatils convenant à l'invention peuvent être les alcanes linéaires comprenant de 8 à 14 atomes de carbone.

De façon préférée, le ou les alcanes linéaires volatils convenant à l'invention peuvent être les alcanes linéaires comprenant de 9 à 14 atomes de carbone.

De façon préférée, le ou les alcanes linéaires volatils convenant à l'invention peuvent être les alcanes linéaires comprenant de 10 à 14 atomes de carbone.

De façon encore plus préférée, le ou les alcanes linéaires volatils convenant à l'invention peuvent être les alcanes linéaires comprenant de 11 à 14 atomes de carbone.

Le ou les alcanes linéaires volatils convenant à l'invention peuvent être avantageusement d'origine végétale.

De préférence, l'alcane linéaire volatil ou le mélange d'alcanes linéaires volatils présent dans la composition utilisée selon l'invention comprend au moins un isotope ¹⁴C du carbone (carbone 14), en particulier l'isotope ¹⁴C peut être présent en un ratio ¹⁴C / ¹²C supérieur ou égal à 1.10⁻¹⁶, de préférence supérieur ou égal à 1.10⁻¹⁵, de préférence encore supérieur ou égal 7,5.10⁻¹⁴, et mieux supérieur ou égal 1,5.10⁻¹³. De préférence, le ratio ¹⁴C/¹²C va de 6.10⁻¹³ à 1,2.10⁻¹² (rapport en nombre d'isotopes).

La quantité d'isotopes ¹⁴C dans l'alcane linéaire volatil ou le mélange d'alcanes linéaires volatils peut être déterminée par des méthodes connues de l'homme du métier telles que la méthode de comptage de Libby, la spectrométrie à scintillation liquide ou encore la spectrométrie de masse à accélération (Accelerator Mass Spectrometry).

Un tel alcane peut être obtenu, directement ou en plusieurs étapes, à partir d'une matière première végétale comme une huile, un beurre, une cire, etc.

A titre d'exemple d'alcanes linéaires volatils convenant à l'invention, on peut mentionner les alcanes décrits dans les demandes de brevets de la société Cognis WO 2007/068371, ou WO2008/155059 (mélanges d'alcanes distincts et différant d'au moins un carbone). Ces alcanes sont obtenus à partir d'alcools gras, eux-mêmes obtenus à partir d'huile de coprah ou de palme.

A titre d'exemple d'alcanes linéaires volatils convenant à l'invention, on peut citer le n- heptane (C₇), le n-octane (C₈), le n-nonane (C₉), le n-décane (C₁₀), le n-undécane (C₁₁), le n-dodécane (C₁₂), le n-tridécane (C₁₃), le n-tétradécane (C₁₄), et leurs mélanges. Selon un mode de réalisation particulier, l'alcane linéaire volatil est choisi parmi le n-nonane, le n-undécane, le n-dodécane, le n-tridécane, le n-tétradécane, et leurs mélanges.

Selon un mode préféré, on peut citer les mélanges de n-undécane (C₁₁) et de n-tridécane (C₁₃) obtenus aux exemples 1 et 2 de la demande WO2008/155059 de la Société Cognis.

On peut également citer le n-dodécane (C₁₂) et le n-tétradécane (C₁₄) vendus par Sasol respectivement sous les références PARAFOL 12-97 et PARAFOL 14-97, ainsi que leurs mélanges.

On pourra utiliser l'alcane linéaire volatil seul.

On pourra alternativement ou préférentiellement utiliser un mélange d'au moins deux alcanes linéaires volatils distincts, différant entre eux d'un nombre de carbone n d'au moins 1, en particulier différant entre eux d'un nombre de carbone de 1 ou de 2.

Selon un mode de réalisation, on utilise un mélange d'au moins deux alcanes linéaires volatils distincts comportant de 10 à 14 atomes de carbone et différant entre eux d'un nombre de carbone d'au moins 1. A titre d'exemples, on peut citer notamment les mélanges d'alcanes linéaires volatils C₁₀/C₁₁, C₁₁/C₁₂, ou C₁₂/C₁₃.

Selon un autre mode de réalisation, on utilise un mélange d'au moins deux alcanes linéaires volatils distincts comportant de 10 à 14 atomes de carbone et différant entre eux d'un nombre de carbone d'au moins 2. A titre d'exemples, on peut citer notamment les mélanges d'alcanes linéaires volatils C₁₀/C₁₂, ou C₁₂/C₁₄, pour un nombre de carbone n pair et le mélange C₁₁/C₁₃ pour un nombre de carbone n impair.

Selon un mode préféré, on utilise un mélange d'au moins deux alcanes linéaires volatils comportant de 10 à 14 atomes de carbone distincts et différant entre eux d'un nombre de carbone d'au moins 2, et en particulier un mélange d'alcanes linéaires volatils C₁₁/C₁₃ ou un mélange d'alcanes linéaires volatils C₁₂/C₁₄.

D'autres mélanges associant plus de 2 alcanes linéaires volatils selon l'invention, tels que par exemple un mélange d'au moins 3 alcanes linéaires volatils comportant de 7 à 15 atomes de carbone distincts et différant entre eux d'un nombre de carbone d'au moins 1, font également partie de l'invention, mais les mélanges de 2 alcanes linéaires volatils selon l'invention sont préférés (mélanges binaires), lesdits 2 alcanes linéaires volatils représentant de préférence plus de 95% et mieux plus de 99% en poids de la teneur totale en alcanes linéaires volatils dans le mélange.

Selon un mode particulier de l'invention, dans un mélange d'alcanes linéaires volatils, l'alcane linéaire volatil ayant le nombre de carbone le plus petit est majoritaire dans le mélange.

Selon un autre mode de l'invention, on utilise un mélange d'alcanes linéaires volatils dans lequel l'alcane linéaire volatil ayant le nombre de carbone le plus grand est majoritaire dans le mélange.

A titre d'exemples de mélanges convenant à l'invention, on peut citer notamment les mélanges suivants :
- de 50 à 90% en poids, de préférence de 55 à 80% en poids, préférentiellement encore de 60 à 75% en poids d'alcane linéaire volatil en Cn avec n allant de 7 à 15,
- de 10 à 50% en poids, de préférence de 20 à 45% en poids, de préférence de 24 à 40% en poids, d'alcane linéaire volatil en Cn+x avec x supérieur ou égal à 1, de préférence x=1 ou x=2, avec n+x compris entre 8 et 14,
par rapport au poids total des alcanes dans ledit mélange.

En particulier, ledit mélange d'alcanes linéaires volatiles peut contenir en outre :
- moins de 2% en poids, de préférence moins de 1% en poids d'hydrocarbures ramifiés,
- et/ou moins de 2% en poids, de préférence moins de 1% en poids d'hydrocarbures aromatiques,
- et/ou moins de 2% en poids, de préférence moins de 1% en poids et préférentiellement moins de 0,1% en poids d'hydrocarbures insaturés dans le mélange.

Plus particulièrement, un alcane linéaire volatil convenant à l'invention peut être mis en oeuvre sous la forme d'un mélange n-undécane/n-tridécane.

En particulier, on utilisera un mélange d'alcanes linéaires volatils comprenant :
- de 55 à 80% en poids, de préférence de 60 à 75% en poids d'alcane linéaire volatil en C11 (n-undécane),
- de 20 à 45% en poids, de préférence de 24 à 40% en poids d'alcane linéaire volatil en C13 (n-tridécane),
par rapport au poids total des alcanes dans ledit mélange.

Selon un mode de réalisation particulier, le mélange d'alcanes est un mélange n-undécane/n-tridécane. En particulier un tel mélange peut être obtenu selon l'exemple 1 ou l'exemple 2 du WO 2008/155059.

Selon un autre mode de réalisation particulier, on utilise le n-dodécane vendu sous la référence PARAFOL 12-97 par SASOL.

Selon un autre mode de réalisation particulier, on utilise le n-tétradécane vendu sous la référence PARAFOL 14-97 par SASOL.

Selon encore un autre mode de réalisation, on utilise un mélange de n-dodécane et de n-tétradécane. On peut utiliser en particulier le mélange dodécane/tétradécane dans le rapport pondéral 85/15 commercialisé par la société BIOSYNTHIS sous la référence VEGELIGHT 1214.

La composition utilisée selon l'invention peut comprendre de 0,5 % à 90 % en poids, en particulier de 1 à 50 % en poids, et plus particulièrement de 1,5 à 40 % en poids et mieux de 2 à 30% en poids d'un ou plusieurs alcanes linéaires volatils par rapport au poids total de la composition.

Le ou les alcanes linéaires volatils forment, seuls ou avec un ou plusieurs autres composés listés ci-dessous, la phase grasse liquide de la composition.

Comme expliqué précédemment, la composition utilisée selon l'invention comprend, outre le ou les alcanes linéaires volatils, un ou plusieurs polymères associatifs non ioniques.

Par polymère, on entend au sens de la présente invention tout composé issu de la polymérisation par polycondensation ou de la polymérisation radicalaire de monomères dont l'un au moins est différent d'un oxyde d'alkylène et d'un composé monofonctionnel de formule RX, R désignant un groupe alkyle ou alkényle en C₁₀-C₃₀, éventuellement hydroxylé, et X désignant un groupement acide carboxylique, amine, amide, hydroxyle, ester. Sont en particulier exclus tous les composés issus uniquement de la simple condensation d'un oxyde d'alkylène sur un alcool gras, un ester gras, un acide gras, un amide gras, une amine grasse.

Il est rappelé que les polymères associatifs sont des polymères amphiphiles capables, dans un milieu aqueux, de s'associer réversiblement entre eux ou avec d'autres molécules. Leur structure chimique comprend plus particulièrement au moins une zone hydrophile et au moins une zone hydrophobe. Par groupement hydrophobe, on entend un radical ou polymère à chaîne hydrocarbonée, saturée ou non, linéaire ou ramifiée, comprenant au moins 8 atomes de carbone, de préférence de 10 à 30 atomes de carbone, en particulier de 12 à 30 atomes de carbone et plus préférentiellement de 18 à 30 atomes de carbone. A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Les polymères associatifs non ioniques sont choisis de préférence parmi :
- (1) les celluloses modifiées par des groupements comportant au moins une chaîne grasse ; on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS (alkyles en C₁₆) vendu par la société AQUALON, ou le produit BERMOCOLL EHM 100 vendu par la société BEROL NOBEL,
   - celles modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL.
- (2) les hydroxypropylguars modifiés par des groupements comportant au moins une chaîne grasse tel que le produit ESAFLOR HM 22 (chaîne alkyle en C₂₂) vendu par la société LAMBERTI, les produits RE210-18 (chaîne alkyle en C₁₄) et RE205-1 (chaîne alkyle en C₂₀) vendus par la société RHODIA.
- (3) les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208.
- (4) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
- (5) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
- (6) les polymères à squelette aminoplaste éther possédant au moins une chaîne grasse, tels que les composés PURE THIX proposés par la société SUD-CHEMIE.
- (7) les copolymères de vinyl pyrrolidone et de monomères hydrophobes à chaîne grasse ; on peut citer à titre d'exemple :
   - les produits ANTARON V216 ou GANEX V216 (copolymère vinylpyrrolidone / hexadécène) vendu par la société I.S.P.
   - les produits ANTARON V220 ou GANEX V220 (copolymère vinylpyrrolidone / eicosène) vendu par la société I.S.P.

De préférence, les polyéthers polyuréthanes comportent au moins deux chaînes lipophiles hydrocarbonées, ayant de 8 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.

Par extension figurent aussi parmi les polyéthers polyuréthanes non-ioniques à chaîne grasse, ceux dont les séquences hydrophiles sont liées aux séquences lipophiles par d'autres liaisons chimiques.

A titre d'exemples de polyéthers polyuréthanes non-ioniques à chaîne grasse, on peut aussi utiliser aussi le Rhéolate 205 à fonction urée vendu par la société RHEOX ou encore les Rhéolates 208, 204 ou 212, ainsi que l'Acrysol RM 184, l'Aculyn 44 et l'Aculyn 46 de la société ROHM & HAAS [l'ACULYN 46 est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44 est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

On peut également citer le produit ELFACOS T210 à chaîne alkyle en C₁₂₋₁₄ et le produit ELFACOS T212 à chaîne alkyle en C₁₈ de chez AKZO.

Le produit DW 1206B de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Rhéolate 255, le Rhéolate 278 et le Rhéolate 244 vendus par la société RHEOX. On peut aussi utiliser le produit DW 1206F et le DW 1206J proposés par la société ROHM & HAAS.

On peut encore utiliser le polyuréthane-39 commercialisé sous la référence Luvigel Star par la société BASF.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Le ou les polymères associatifs non ioniques sont de préférence choisis parmi les polyuréthanes polyéthers.

Le ou le(s) polymère(s) associatif(s) non ioniques sont de préférence présents en une quantité allant de 0,05 à 10% en poids, mieux encore de 0,1 à 5% en poids, et encore plus préférentiellement de 0,2 à 2% en poids par rapport au poids total de la composition.

La composition utilisée selon l'invention peut comprendre en outre un ou plusieurs corps gras non siliconés.

De préférence, le ou les corps gras non siliconés sont choisis parmi les alcools gras, les acides gras, les esters d'acide gras et d'alcool gras, les cires, les huiles végétales, animales, minérales et synthétiques.

Les alcools gras peuvent être choisis parmi les alcools de formule R'OH, où R' désigne un radical saturé ou insaturé, linéaire ou ramifié, comportant de préférence de 8 à 40 atomes de carbone, de préférence 8 à 30 atomes de carbone. R' désigne de préférence un groupement alkyle en C₁₂-C₂₄ ou alkényle en C₁₂-C₂₄. R peut être substitué par un ou plusieurs groupements hydroxy.

Les alcools gras peuvent être en particulier choisis parmi l'alcool laurique, l'alcool cétylique, l'alcool dodécylique, l'alcool décylique, l'alcool stéarylique, l'alcool oléïque, l'alcool béhénique, l'alcool linoléique, l'alcool undécylénique, l'alcool palmitoléïque, l'alcool arachidonique, l'alcool myristylique et l'alcool érucique. On peut également utiliser un mélange d'alcools gras, ce qui signifie que dans un produit commercial peuvent coexister plusieurs espèces d'alcools gras, sous forme d'un mélange. A titre de mélange d'alcools gras, on peut citer l'alcool cétylstéarylique ou cétéarylique.

Les acides gras peuvent être choisis parmi les acides de formule RCOOH, où R est un radical saturé ou insaturé, linéaire ou ramifié, comportant de préférence de 7 à 39 atomes de carbone.

De préférence, R est un groupement alkyle en C₇-C₂₉ ou alkényle en C₇-C₂₉, mieux un groupement alkyle en C₁₂-C₂₄ ou alkényle en C₁₂-C₂₄. R peut être substitué par un ou plusieurs groupements hydroxy et/ou un ou plusieurs groupe carboxyle.

L'acide gras de l'ester peut être en particulier choisi parmi l'acide laurique, l'acide oléique, l'acide palmitique, l'acide linoléique, l'acide myristique et l'acide stéarique.

Les cires sont des substances naturelles (animales ou végétales) ou synthétiques solides à température ambiante (20°-25°C). Elles sont insolubles dans l'eau, solubles dans les huiles et sont capables de former un film hydrofuge.

Sur la définition des cires, on peut citer par exemple P.D. Dorgan, Drug and Cosmetic Industry, Decembre 1983, pp. 30-33.

La cire ou les cires éventuellement présentes dans la composition utilisée selon l'invention peuvent être choisies notamment, parmi la cire de Carnauba, la cire de Candelilla, la cire d'Alfa, la cire de paraffine, l'ozokérite, les cires végétales telles que la cire d'olivier, la cire de riz, la cire de jojoba hydrogénée ou les cires absolues de fleurs telles que la cire essentielle de fleur de cassis, les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines.

A titre d'huile végétale, on peut citer l'huile de jojoba, l'huile d'avocat, l'huile de colza, l'huile d'olive, l'huile de tournesol, l'huile de maïs, l'huile de soja, l'huile de courge, l'huile de pépins de raisin, l'huile de sésame, l'huile de noisette, l'huile d'abricot, l'huile de macadamia, l'huile d'arara, l'huile de tournesol, l'huile de ricin.

Lorsqu'il sont présents, le ou les corps gras non siliconés représentent généralement de 0,5 à 20% en poids du poids total de la composition.

La composition utilisée selon l'invention peut également comprendre une ou plusieurs silicones.

Les silicones éventuellement présentes dans la composition utilisée selon l'invention sont en particulier des polyorganosiloxanes qui peuvent se présenter sous forme de solutions aqueuses, c'est-à-dire solubilisées, ou éventuellement sous forme de dispersions ou micro-dispersions, ou d'émulsions aqueuses. Les polyorganosiloxanes peuvent également se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press.

Les silicones peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5.
   Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHONE POULENC, le décaméthylcyclopentasiloxane commercialisé sous le nom de "VOLATILE SILICONE 7158" par UNION CARBIDE, "SILBIONE 70045 V 5" par RHONE POULENC, ainsi que leurs mélanges.
   On peut également citer les cyclocopolymères du type diméthylsiloxanes/méthylalkylsiloxane, tel que la "SILICONE VOLATILE FZ 3109" commercialisée par la société UNION CARBIDE, de structure chimique : 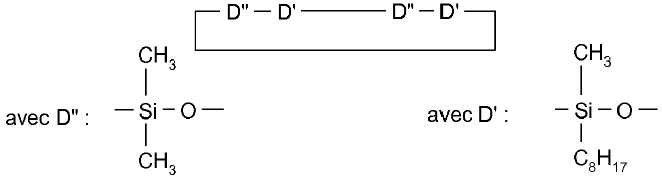 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle (Diméthicone selon la dénomination CTFA) ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC.

On peut également citer les polydiméthylsiloxanes à groupements aminoéthyl aminopropyl et alpha-oméga silanols.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles SILBIONE de la série 70 641 de RHONE POULENC ;
- les huiles des séries RHODORSIL 70 633 et 763 de RHONE POULENC ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone pouvant être présentes dans la composition utilisée selon l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- les gommes de polydiméthylsiloxane,
- les gommes polydiméthylsiloxanes/méthylvinylsiloxane,
- les gommes de polydiméthylsiloxane/diphénylsiloxane,
- les gommes de polydiméthylsiloxane/phénylméthylsiloxane,
- les gommes de polydiméthylsiloxane / diphénylsiloxane/ méthylvinylsiloxane.

Des produits plus particulièrement utilisables sont les mélanges suivants:
- les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻6m²/s. Ce produit comporte de préférence 15 % de gomme SE 30 et 85 % d'une huile SF 96.

Les résines d'organopolysiloxanes éventuellement présentes dans la composition utilisée selon l'invention sont des systèmes siloxaniques réticulés renfermant les unités : R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées éventuellement présentes dans la composition utilisée selon l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 ;
- des groupements acyloxyalkyle tels que par exemple les polyorganosiloxanes décrits dans le brevet US-A-4957732 ;
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255" ;
- des groupements hydroxyacylamino, comme les polyorganosiloxanes décrits dans la demande EP 342 834. On peut citer par exemple le produit Q2-8413 de la société DOW CORNING.

Parmi les silicones organomodifiées, on peut encore citer les silicones aminées.

Par silicone aminée, on entend toute silicone comportant au moins une fonction amine primaire, secondaire, tertiaire ou un groupement ammonium quaternaire.

Les silicones aminées éventuellement utilisées dans la composition cosmétique selon la présente invention sont choisies parmi :
(a) les composés répondant à la formule (I) suivante :

   (R¹)ₐ(T)₃₋ₐ-Si[OSi(T)₂]ₙ-[OSi(T)_{b}(R¹)_{2-b]m}-OSi(_{T})₃₋ₐ-(R¹)ₐ (I)

   dans laquelle,
   T est un atome d'hydrogène, ou un radical phényle, hydroxyle (-OH), ou alkyle en C₁-C₈, et de préférence méthyle ou alcoxy en C₁-C₈, de préférence méthoxy,
   a désigne le nombre 0 ou un nombre entier de 1 à 3, et de préférence 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R₁ est un radical monovalent de formule -C_{q}H_{2q}L dans laquelle q est un nombre de 2 à 8 et L est un groupement aminé éventuellement quaternisé choisi parmi les groupements :
      -N(R²)-CH₂-CH₂-N(R²)₂ ;
      -N(R²)₂ ; -N⁺(R²)₃ Q- ;
      -N⁺(R²) (H)₂ Q- ;
      -N⁺(R²)₂HQ- ;
      -N(R²)-CH₂-CH₂-N⁺(R²)(H)₂ Q-,
   dans lesquels R² peut désigner un atome d'hydrogène, un phényle, un benzyle, ou un radical hydrocarboné saturé monovalent, par exemple un radical alkyle en C₁-C₂₀, et Q- représente un ion halogénure tel que par exemple fluorure, chlorure, bromure ou iodure.
   En particulier, les silicones aminées correspondant à la définition de la formule (I) sont choisies parmi les composés correspondant à la formule suivante : 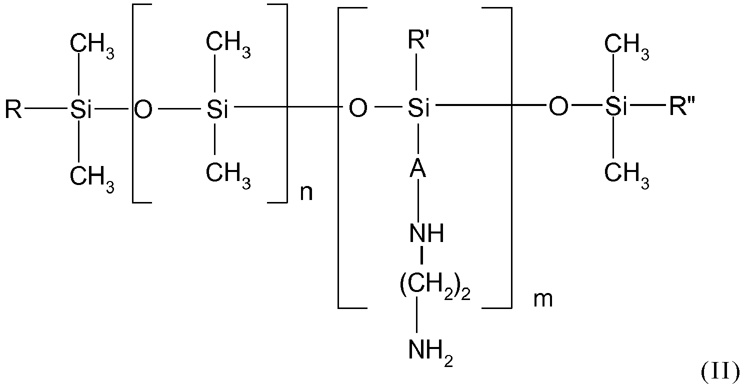 dans laquelle R, R', R", identiques ou différents, désignent un radical alkyle en C₁-C₄, de préférence CH₃ ; un radical alcoxy en C₁-C₄, de préférence méthoxy ; ou OH ; A représente un radical alkylène, linéaire ou ramifié, en C₃-C₈, de préférence en C₃-C₆; m et n sont des nombres entiers dépendant du poids moléculaire et dont la somme est comprise entre 1 et 2000.
   Selon une première possibilité, R, R', R", identiques ou différents, représentent un radical alkyle en C₁-C₄ ou hydroxyle, A représente un radical alkylène en C₃ et m et n sont tels que la masse moléculaire moyenne en poids du composé est comprise entre 5 000 et 500 000 environ. Les composés de ce type sont dénommés dans le dictionnaire CTFA, "amodiméthicone".
   Selon une deuxième possibilité, R, R', R", identiques ou différents, représentent un radical alcoxy en C₁-C₄ ou hydroxyle, l'un au moins des radicaux R ou R" est un radical alcoxy et A représente un radical alkylène en C₃. Le rapport molaire hydroxy / alcoxy est de préférence compris entre 0,2/1 et 0,4/1 et avantageusement égal à 0,3/1. Par ailleurs, m et n sont tels que la masse moléculaire moyenne en poids du composé est comprise entre 2000 et 10⁶. Plus particulièrement, n est compris entre 0 et 999 et m est compris entre 1 et 1000, la somme de n et m étant comprise entre 1 et 1000.
   Dans cette catégorie de composés, on peut citer entre autres, le produit Belsil®ADM 652, commercialisée par Wacker.
   Selon une troisième possibilité, R, R", différents, représentent un radical alcoxy en C₁-C₄ ou hydroxyle, l'un au moins des radicaux R, R" est un radical alcoxy, R' représente un radical méthyle et A représente un radical alkylène en C₃. Le rapport molaire hydroxy / alcoxy est de préférence compris entre 1/0,8 et 1/1,1, et avantageusement est égal à 1/0,95. Par ailleurs, m et n sont tels que la masse moléculaire moyenne en poids du composé est comprise entre 2000 et 200000. Plus particulièrement, n est compris entre 0 et 999 et m est compris entre 1 et 1000, la somme de n et m étant comprise entre 1 et 1000.
   Plus particulièrement, on peut citer le produit FluidWR® 1300, commercialisé par Wacker.
   Notons que la masse moléculaire de ces silicones est déterminée par chromatographie par perméation de gel (température ambiante, étalon polystyrène ; colonnes µ styragem ; éluant THF ; débit de 1 mm/m ; on injecte 200 µl d'une solution à 0,5 % en poids de silicone dans le THF et l'on effectue la détection par réfractométrie et UV-métrie).
   Un produit correspondant à la définition de la formule (I) est en particulier le polymère dénommé dans le dictionnaire CTFA "triméthylsilylamodiméthicone", répondant à la formule (III) suivante: 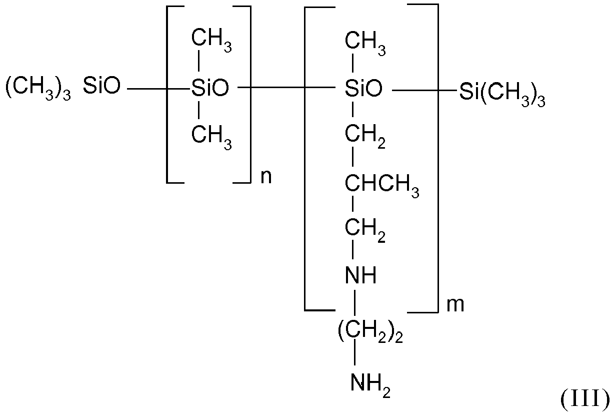 dans laquelle n et m ont les significations données ci-dessus conformément à la formule (I).
   De tels composés sont décrits par exemple dans EP 95238; un composé de formule (III) est par exemple vendu sous la dénomination Q2-8220 par la société OSI.
(b) les composés répondant à la formule (IV) suivante : 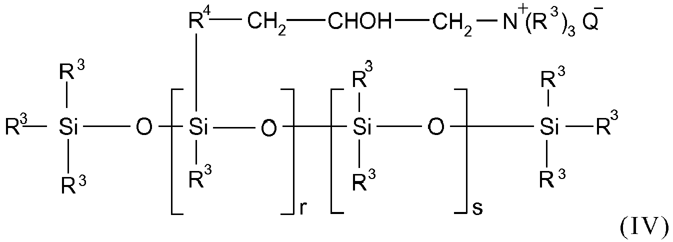 dans laquelle,
   R³ représente un radical hydrocarboné monovalent en C₁-C₁₈, et en particulier un radical alkyle en C₁-C₁₈, ou alcényle en C₂-C₁₈, par exemple méthyle ;
   R⁴ représente un radical hydrocarboné divalent, notamment un radical alkylène en C₁-C₁₈ ou un radical alkylèneoxy divalent en C₁-C₁₈, par exemple en C₁-C₈ ;
   Q⁻ est un ion halogénure, notamment chlorure ;
   r représente une valeur statistique moyenne de 2 à 20 et en particulier de 2 à 8 ;
   s représente une valeur statistique moyenne de 20 à 200 et en particulier de 20 à 50.
   De tels composés sont décrits plus particulièrement dans le brevet US 4185087.
   Un composé entrant dans cette classe est celui vendu par la Société Union Carbide sous la dénomination "Ucar Silicone ALE 56".
c) les silicones ammonium quaternaire de formule (V) : 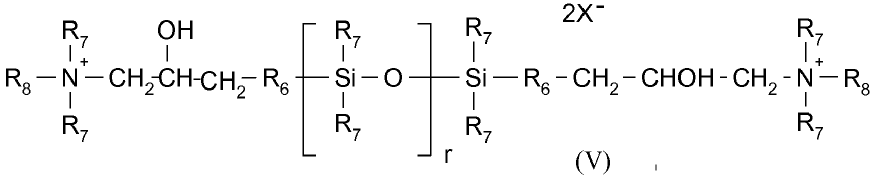 dans laquelle :
   R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
   R₆ représente un radical hydrocarboné divalent, notamment un radical alkylène en C₁-C₁ ou un radical alkylèneoxy divalent en C₁-C₁₈, par exemple en C₁-C₈ relié au Si par une liaison SiC ;
   R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈ , un radical -R₆-NHCOR₇,
   X- est un anion tel qu'un ion halogénure, notamment chlorure ou un sel d'acide organique (acétate ...);
   r représente une valeur statistique moyenne de 2 à 200 et en particulier de 5 à 100 ;
   Ces silicones sont par exemple décrites dans la demande EP-A-0530974.
d) les silicones aminées de formule (VI) : 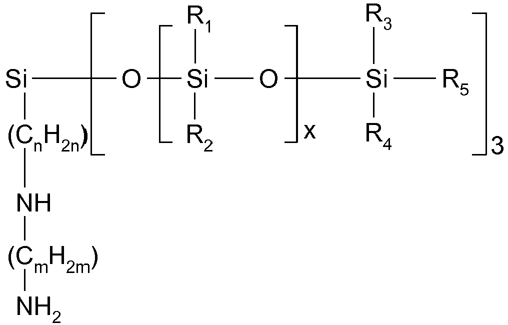 dans laquelle :
   - R₁, R₂, R³ et R₄, identiques ou différents, désignent un radical alkyle en C₁-C₄ ou un groupement phényle,
   - R₅ désigne un radical alkyle en C₁-C₄ ou un groupement hydroxyle,
   - n est un entier variant de 1 à 5,
   - m est un entier variant de 1 à 5,
   et dans laquelle x est choisi de manière telle que l'indice d'amine soit compris entre 0,01 et 1 meq/g.

Les silicones particulièrement préférées sont les polydiméthylsiloxanes, les diméthicones et les amodiméthicones.

Lorsque ces composés sont mis en oeuvre, une forme de réalisation particulièrement intéressante est leur utilisation conjointe avec des agents de surface cationiques et/ou non ioniques.

A titre exemple, on peut utiliser le produit vendu sous la dénomination "Emulsion Cationique DC 939" par la Société Dow Corning, qui comprend, outre l'amodiméthicone, un agent de surface cationique qui est le chlorure de triméthylcétylammonium et un agent de surface non ionique de formule : C₁₃H₂₇-(OC₂H₄)₁₂-OH, connu sous la dénomination CTFA "tridéceth-12".

Un autre produit commercial utilisable selon l'invention est le produit vendu sous la dénomination "Dow Corning Q2 7224" par la Société Dow Corning, comportant en association le triméthylsilylamodiméthicone de formule (III) décrite ci-dessus, un agent de surface non ionique de formule : C₈H₁₇-C₆H₄-(OCH₂CH₂)₄₀-OH, connu sous la dénomination CTFA "octoxynol-40", un second agent de surface non ionique de formule: C₁₂H₂₅-(OCH₂-CH₂)₆-OH, connu sous la dénomination CTFA "isolaureth-6", et du propylèneglycol.

La ou les silicones représentent généralement de 0,1 à 20%, de préférence de 0,1 à 10%, en poids du poids total de la composition.

La composition cosmétique utilisée selon l'invention peut également comprendre un ou plusieurs polymères différents des polymères associatifs non ioniques décrits ci-dessus.

Le ou les polymères différents des polymères associatifs non ioniques décrits ci-dessus peuvent être d'origine naturelle, végétale ou minérale, et/ou de synthèse.

Les polymères d'origine naturelle peuvent être choisis parmi les pectines, les celluloses, les alginates, le galactoarabinane, la gomme adragante, les amidons et le saccharose.

Les polymères d'origine végétale et modifiés par voie de synthèse peuvent être choisis par exemple parmi les dérivés d'amidon, tels que carboxyméthylamidon et le phosphate de diamidon, et les dérivés de cellulose tels que l'hydroxyéthylcellulose et la carboxyméthylcellulose.

Les polymères peuvent être choisis parmi les polymères cationiques, anioniques, amphotères et non ioniques.

Les polymères cationiques sont tout particulièrement intéressants en tant qu'agents de conditionnement des fibres kératiniques.

Par « polymère cationique », on entend au sens de la présente invention, tout polymère comprenant des groupements cationiques et/ou des groupements ionisables en groupements cationiques.

Les polymères cationiques utilisables dans la composition cosmétique utilisée selon l'invention sont de préférence choisis parmi les polymères comportant des groupements amine primaire, secondaire, tertiaire et/ou quaternaire faisant partie de la chaîne polymère ou directement reliés à celle-ci, et ayant une masse moléculaire moyenne en nombre comprise entre 500 et environ 5 000 000, et de préférence entre 1 000 et 3 000 000.

Parmi ces polymères, on peut citer plus particulièrement les polymères cationiques suivants :
(1) les homopolymères ou copolymères d'esters ou d'amides acryliques ou méthacryliques, à fonctions aminées, comportant au moins un des motifs de formules suivantes : 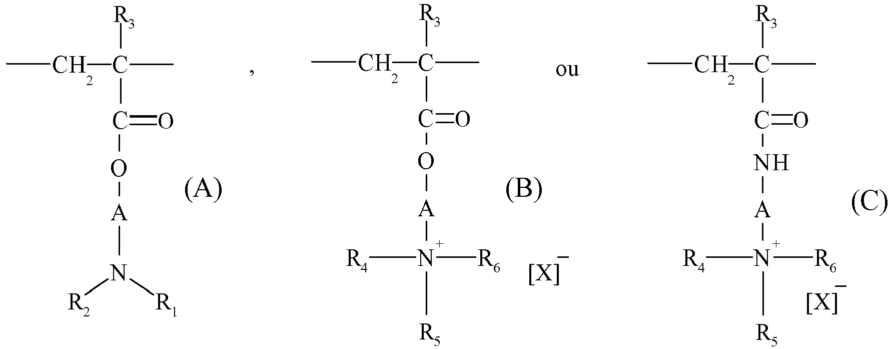 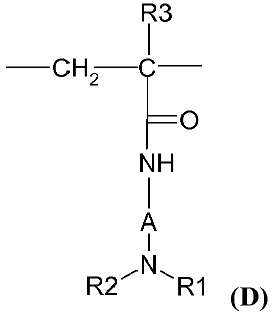 dans lesquelles:
   R₁ et R₂, identiques ou différents, représentent chacun un atome d'hydrogène ou un groupe alkyle ayant de 1 à 6 atomes de carbone ;
   R³ désigne un atome d'hydrogène ou un groupe CH₃ ;
   A est un groupe alkyle linéaire ou ramifié, comportant de 1 à 6 atomes de carbone ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle ;
   X désigne un anion méthosulfate ou un halogénure tel que chlorure ou bromure.
   Les copolymères de la famille (1) contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieur (C₁₋₄), des groupes dérivés des acides acryliques ou méthacryliques ou de leurs esters, de vinyllactames tels que la vinylpyrrolidone ou le vinylcaprolactame, d'esters vinyliques.
   Ainsi, parmi ces copolymères de la famille (1), on peut citer :
   - les copolymères d'acrylamide et de méthacrylate de diméthylaminoéthyle quaternisés au sulfate de diméthyle ou avec un halogénure de diméthyle, tels que celui vendu sous la dénomination HERCOFLOC^{®} par la société HERCULES,
   - les copolymères d'acrylamide et de chlorure de méthacryloyloxyéthyltriméthylammonium décrits, par exemple, dans la demande de brevet EP-A-080976 et vendus sous la dénomination BINA QUAT P 100 par la société CIBA GEIGY,
   - les copolymères d'acrylamide et de méthosulfate de méthacryloyloxyéthyltriméthylammonium, tels que celui vendu sous la dénomination RETEN par la société HERCULES,
   - les copolymères vinylpyrrolidone/acrylate ou méthacrylate de dialkylaminoalkyle quaternisés ou non, tels que les produits vendus sous la dénomination "GAFQUAT^{®}" par la société ISP comme, par exemple, "GAFQUAT^{®} 734" ou "GAFQUAT^{®} 755", ou bien les produits dénommés "COPOLYMER^{®} 845, 958 et 937". Ces polymères sont décrits en détail dans les brevets français Nos 2 077 143 et 2 393 573,
   - les terpolymères méthacrylate de diméthylaminoéthyle/ vinylcaprolactame/vinylpyrrolidone tels que le produit commercialisé sous la dénomination GAFFIX^{®} VC 713 par la société ISP, et
   - les copolymères vinylpyrrolidone/méthacrylamide de diméthylaminopropyle quaternisé tels que notamment le produit commercialisé sous la dénomination "GAFQUAT^{®} HS 100" par la société ISP ;
   - les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la SociétéCIBA.
(2) les polysaccharides cationiques, et en particulier ceux choisis parmi
   a) les dérivés d'éthers de cellulose comportant des groupements ammonium quaternaire décrits dans le brevet FR 1492597 et en particulier les polymères commercialisés sous les dénominations "UCARE POLYMER JR" (JR 400 LT, JR 125, JR 30M) ou "LR" (LR 400, LR 30M) par la Société AMERCHOL. Ces polymères sont également définis dans le dictionnaire CTFA comme des ammoniums quaternaires d'hydroxyéthyl cellulose ayant réagi avec un époxyde substitué par un groupement triméthylammonium ;
   b) les dérivés de cellulose greffés avec un monomère hydrosoluble comportant un ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyloxyéthyl-triméthylammonium, de méthacrylamidopropyl triméthylammonium, de diméthyl-diallylammonium.
      Les produits commercialisés répondant à cette définition sont plus particulièrement les produits vendus sous la dénomination "CELQUAT L 200" et "CELQUAT H 100" par la Société National Starch.
   c) les polygalactomananes cationiques tels que ceux décrits dans les brevets américains 3 589 578 et 4 031 307 tel que les gommes de guar contenant des groupements cationiques trialkylammonium. De tels produits sont commercialisés notamment sous les dénominations commerciales de JAGUAR C13 S, JAGUAR C 15, JAGUAR C 17 par la société RHODIA ;
(3) Les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2 162 025 et 2 280 361.
(4) Les polyaminoamides solubles dans l'eau, préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alkylés ou s'ils comportent une ou plusieurs fonctions amines tertiaires, quaternisées. De tels polymères sont notamment décrits dans les brevets français 2 252 840 et 2 368 508.
(5) Les dérivés de polyaminoamides résultant de la condensation de polyalkylènes-polyamines avec des acides polycarboxyliques, suivie d'une alkylation par des agents bifonctionnels. On peut citer, par exemple, les polymères acide adipique/diakylaminohydroxyalkyl-dialkylènetriamine dans lesquels le groupe alkyle comporte de 1 à 4 atomes de carbone et désigne de préférence un groupe méthyle, éthyle, propyle, et le groupe alkylène comporte de 1 à 4 atomes de carbone, et désigne de préférence le groupe éthylène. De tels polymères sont notamment décrits dans le brevet français 1 583 363.
   Parmi ces dérivés, on peut citer plus particulièrement les polymères acide adipique/diméthylaminohydroxypropyl-diéthylène-triamine.
(6) Les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone. Le rapport molaire entre la polyalkylène-polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3 227 615 et 2 961 347.
(7) Les cyclopolymères d'alkyldiallylamine ou de dialkyldiallylammonium tels que les homopolymères ou copolymères comportant, comme constituant principal de la chaîne, des motifs répondant aux formules (VIIa) ou (VIIb) : 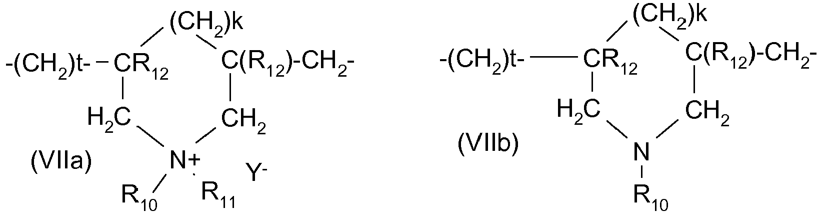 dans lesquelles k et t sont égaux à 0 ou 1, la somme k + t étant égale à 1 ; R₁₂ désigne un atome d'hydrogène ou un groupe méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou alors R₁₀ et R₁₁ peuvent désigner conjointement avec l'atome d'azote auquel ils sont rattachés, des groupements hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2 080 759 et dans son certificat d'addition 2 190 406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallyl-ammonium vendu sous la dénomination "MERQUAT^{®} 100" par la société NALCO COMPANY (et ses homologues de faibles masses moléculaires moyenne en poids) et les copolymères de chlorure de diallyldiméthylammonium et d'acrylamide commercialisés sous la dénomination "MERQUAT^{®} 550" ou "MERQUAT^{®} 7SPR".
(8) Les polymères de diammonium quaternaire contenant des motifs récurrents répondant à la formule (VIII) : 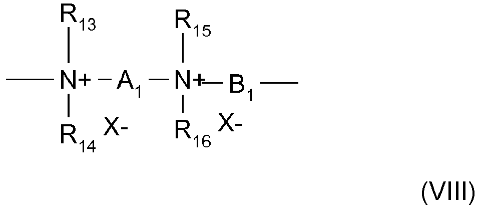 dans laquelle :
   R₁₃, R₁₄, R₁₅ et R₁₆, identiques ou différents, représentent des groupes aliphatiques, alicycliques ou arylaliphatiques contenant de 1 à 20 atomes de carbone ou des groupes hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote, ou bien R₁₃, R₁₄, R₁₅ et R₁₆ représentent un groupe alkyle en C₁-C₆, linéaire ou ramifié, substitué par un groupement nitrile, ester, acyle, amide ou -CO-O-R₁₇-E ou -CO-NH-R₁₇-E où R₁₇ est un groupe alkylène et E un groupement ammonium quaternaire ;
   A₁ et B₁ représentent des groupements polyméthyléniques contenant de 2 à 20 atomes de carbone, pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A₁, R₁₃ et R₁₅ peuvent former avec les deux atomes d'azote auxquels ils sont rattachés un cycle pipérazinique ; en outre, si A₁ désigne un groupe alkylène ou hydroxyalkylène linéaire ou ramifié, saturé ou insaturé, B₁ peut également désigner un groupement :

      -(CH₂)ₙ-CO-E'-OC-(CH₂)n-

      dans lequel E' désigne :
      a) un reste de glycol de formule -O-Z-O-, où Z désigne un groupe hydrocarboné linéaire ou ramifié, ou un groupement répondant à l'une des formules suivantes :

         -(CH₂-CH₂-O)ₓ-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ; ou
      b) un reste de diamine bis-secondaire tel qu'un dérivé de pipérazine ; ou
      c) un reste de diamine bis-primaire de formule -NH-Y-NH-, où Y désigne un groupe hydrocarboné linéaire ou ramifié, ou bien le groupe divalent -CH₂-CH₂-S-S-CH₂-CH₂- ; ou
      d) un groupement uréylène de formule -NH-CO-NH- .
   De préférence, X⁻ est un anion tel que le chlorure ou le bromure.
   Des polymères de ce type sont notamment décrits dans les brevets français 2 320 330, 2 270 846, 2 316 271, 2 336 434 et 2 413 907 et les brevets US 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020.
   On peut utiliser plus particulièrement les polymères qui sont constitués de motifs récurrents répondant à la formule (IX): 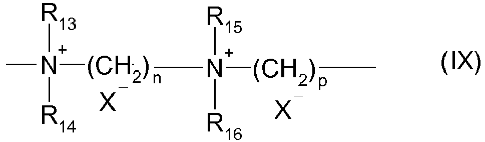 dans laquelle R₁₃, R₁₄, R₁₅ et R₁₆, identiques ou différents, désignent un groupe alkyle ou hydroxyalkyle ayant de 1 à 4 atomes de carbone environ, n et p sont des nombres entiers variant de 2 à 20 environ et, X⁻ est un anion dérivé d'un acide minéral ou organique.
(9) Les polymères de polyammonium quaternaire constitués de motifs de formule (X) : 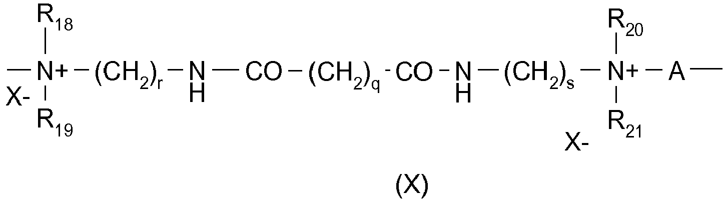 dans laquelle :
   R₁₈, R₁₉, R₂₀ et R₂₁, identiques ou différents, représentent un atome d'hydrogène ou un groupe méthyle, éthyle, propyle, β-hydroxyéthyle, β-hydroxypropyle ou -CH₂CH₂(OCH₂CH₂)ₚOH, où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X⁻ désigne un anion tel qu'un halogénure,
   A désigne un radical d'un dihalogénure ou représente de préférence -CH₂-CH₂-O-CH₂-CH₂-.
   De tels composés sont notamment décrits dans la demande de brevet EP-A-122 324.
(10) Les polymères quaternaires de vinylpyrrolidone et de vinylimidazole tels que, par exemple, les produits commercialisés sous les dénominations Luviquat^{®} FC 905, FC 550 et FC 370 par la société B.A.S.F.
(11) les chitosanes ou leurs sels ; les sels utilisables sont en particulier l'acétate, le lactate, le glutamate, le gluconate ou le pyrrolidone-carboxylate de chitosane.

Parmi ces composés, on peut citer le chitosane ayant un taux de désacétylation de 90,5 % en poids vendu sous la dénomination KYTAN BRUT STANDARD par la société ABER TECHNOLOGIES, le pyrrolidone-carboxylate de chitosane commercialisé sous la dénomination KYTAMER^{®} PC par la société AMERCHOL.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

On peut également utiliser des mélanges de polymères.

Les polymères cationiques des familles (1) et (7) sont particulièrement préférés.

La composition utilisée selon l'invention peut également comprendre à titre de polymères un ou plusieurs amidons modifiés ou non.

Le ou les amidons éventuellement présents dans la composition utilisée selon l'invention sont plus particulièrement des macromolécules sous forme de polymères constitués de motifs élémentaires qui sont des unités anhydroglucose. Le nombre de ces motifs et leur assemblage permettent de distinguer l'amylose (polymère linéaire) et l'amylopectine (polymère ramifié). Les proportions relatives d'amylose et d'amylopectine, ainsi que leur degré de polymérisation, varient en fonction de l'origine végétale des amidons.

Les molécules d'amidons éventuellement utilisés dans la présente invention peuvent provenir d'une source végétale telle que les céréales, les tubercules, les racines, les légumes et les fruits. Ainsi, le ou les amidons peuvent provenir d'une source végétale choisie parmi le maïs, les pois, la pomme de terre, la patate douce, la banane, l'orge, le blé, le riz, l'avoine, le sagou, le tapioca et le sorgo. L'amidon est de préférence issu de la pomme de terre.

On peut également utiliser les hydrolysats des amidons cités ci-dessus.

Les amidons se présentent généralement sous la forme d'une poudre blanche, insoluble dans l'eau froide, dont la taille des particules élémentaires va de 3 à 100 microns.

Les amidons éventuellement utilisés dans la composition utilisée selon l'invention peuvent être modifiés chimiquement par une ou plusieurs des réactions suivantes : prégélatinisation, oxydation, réticulation, estérification, traitements thermiques.

De manière plus particulière, ces réactions peuvent être réalisées de la façon suivante :
- prégélatinisation en faisant éclater les granules d'amidon (par exemple séchage et cuisson dans un tambour sécheur) ;
- oxydation par des oxydants forts conduisant à l'introduction de groupes carboxyle dans la molécule d'amidon et à la dépolymérisation de la molécule d'amidon (par exemple en traitant une solution aqueuse d'amidon par l'hypochlorite de sodium) ;
- réticulation par des agents fonctionnels capables de réagir avec les groupes hydroxyle des molécules d'amidon qui vont ainsi être liées entre elles (par exemple avec des groupes glycéryl et/ou phosphate) ;
- estérification en milieu alcalin pour le greffage de groupes fonctionnels, notamment acyl en C₁-C₆ (acétyl), hydroxyalkylés en C₁-C₆ (hydroxyéthyl, hydroxypropyl), carboxyalkyl (en particulier carboxyméthyl), octénylsuccinique. On peut citer en particulier les amidons modifiés par le carboxyméthyl de sodium.

On peut notamment obtenir par réticulation avec des composés phosphorés des phosphates de monoamidon (du type Am-O-PO-(OX)2), des phosphates de diamidon ( du type Am-O-PO-(OX)-O-Am) ou même de triamidon (du type Am-O-PO- (O-Am)2) ou leurs mélanges.

X désigne notamment les métaux alcalins (par exemple sodium ou potassium), les métaux alcalinoterreux (par exemple calcium, magnésium), les sels d'ammoniaque, les sels d'amines comme ceux de la monoéthanolamine, la diéthanolamine, la triéthanolamine, l'amino-3 propanediol-1,2, les sels ammoniums issus des aminoacides basiques tels que la lysine, l'arginine, la sarcosine, l'ornithine, la citrulline.

Les composés phosphorés peuvent être par exemple du tripolyphosphate de sodium, de l'orthophosphate de sodium, de l'oxychlorure de phosphore ou du trimétaphosphate de sodium.

On utilisera préférentiellement des phosphates de diamidon, en particulier hydroxypropylé, ou des composés riches en phosphate de diamidon, en particulier hydroxypropylé, comme le produit proposé sous les références PREJEL VA-70-T AGGL (phosphate de diamidon de manioc hydroxypropylé gélatinisé) ou PREJEL TK1 (phosphate de diamidon de manioc gélatinisé) ou PREJEL 200 (phosphate de diamidon de manioc acétylé gélatinisé) par la Société AVEBE ou STRUCTURE ZEA de NATIONAL STARCH (phosphate de diamidon de maïs hydroxypropylé gélatinisé).

Lorsque les amidons sont modifiés chimiquement par une réaction d'estérification, on peut obtenir des carboxyalkylamidons, comme indiqué précédemment.

Les carboxyalkylamidons sont de préférence des carboxyalkyl (C₁-C₄) amidon et plus particulièrement des carboxyméthylamidons.

Les sels sont notamment des sels de métal alcalin ou alcalinoterreux tels que Na, K 1/2, Li, NH₄, d'un ammonium quaternaire ou d'une amine organique telle que la mono, di ou triéthanolamine.

Les carboxyalkylamidons sont obtenus par greffage de groupements carboxyalkyl sur une ou plusieurs fonctions alcools de l'amidon, notamment par réaction d'amidon et de monochloroacétate de sodium en milieu alcalin.

Les groupements carboxyalkyl sont généralement fixés par l'intermédiaire d'une fonction éther, plus particulièrement sur le carbone 1.

Le degré de substitution va de préférence de 0,1 à 1 et plus particulièrement de 0,15 à 0,5. Le degré de substitution est défini selon la présente invention comme étant le nombre moyen de groupements hydroxyles substitués par un groupement ester ou éther (en l'occurrence éther pour les carboxyméthylamidons) par unité monosaccharidique du polysaccharide.

Les carboxyalkylamidons comprennent de préférence des motifs de formule suivante:

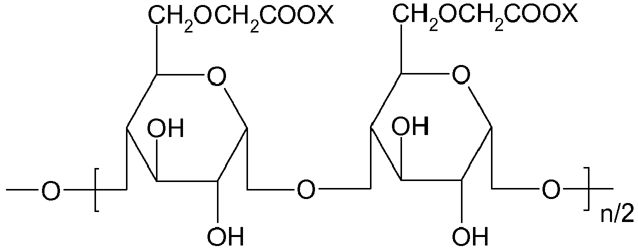

X désigne un atome d'hydrogène, un métal alcalin ou alcalinoterreux tels que Na, K 1/2, Li, NH₄, un ammonium quaternaire ou une amine organique. De préférence X désigne un ion Na+.

Les carboxyalkylamidons utilisables selon la présente invention sont de préférence les carboxyalkylamidons non prégélatinisés.

Les carboxyalkylamidons utilisables selon la présente invention sont de préférence les carboxyalkylamidons réticulés partiellement ou totalement.

Les carboxyalkylamidons utilisables selon la présente invention sont de préférence des sels de sodium de carboxyalkylamidons, en particulier un sel de sodium de carboxyméthylamidon de pomme de terre vendus notamment sous la dénomination PRIMOJEL par la société DMV International. Plus de 95% des particules de cet amidon ont un diamètre inférieur à 100 microns et plus particulièrement inférieur à 65 microns.

Selon l'invention, on peut aussi utiliser des amidons amphotères, ces amidons amphotères contiennent un ou plusieurs groupements anioniques et un ou plusieurs groupements cationiques. Les groupements anioniques et cationiques peuvent être liés au même site réactif de la molécule d'amidon ou à des sites réactifs différents; de préférence ils sont liés au même site réactif. Les groupements anioniques peuvent être de type carboxylique, phosphate ou sulfate et de préférence carboxylique. Les groupements cationiques peuvent être de type amine primaire, secondaire, tertiaire ou quaternaire.

Les amidons amphotères sont notamment choisis parmi les composés de formules suivantes : 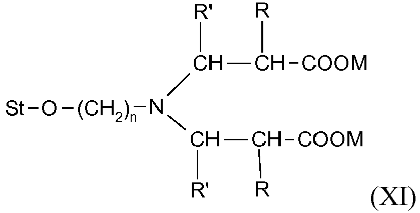 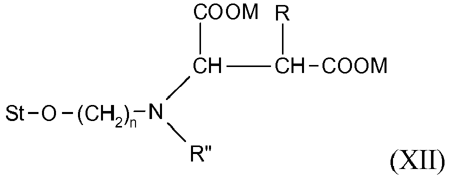 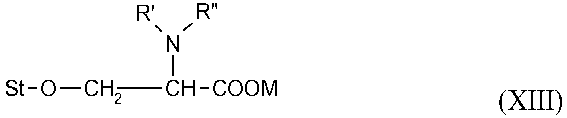 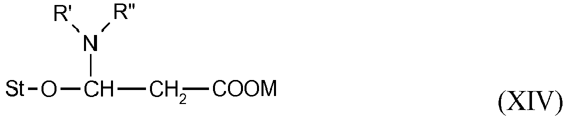 formules dans lesquelles :
St-O représente une molécule d'amidon,
R, identique ou différent, représente un atome d'hydrogène ou un radical méthyle,
R', identique ou différent, représente un atome d'hydrogène, un radical méthyle ou un groupement -COOH,
n est un entier égal à 2 ou 3,
M, identique ou différent, désigne un atome d'hydrogène, un métal alcalin ou alcalinoterreux tels que Na, K, Li, NH₄, un ammonium quaternaire ou une amine organique,
R" représente un atome d'hydrogène ou un radical alkyle ayant de 1 à 18 atomes de carbone.

Ces composés sont notamment décrits dans les brevets US 5,455,340 et US 4,017,460 qui sont inclus à titre de référence.

On utilise particulièrement les amidons de formules (XI) ou (XII). On utilise plus particulièrement les amidons modifiés par de l'acide 2-chloroéthyl aminodipropionique, c'est à dire les amidons de formule (XI) ou (XII) dans lesquelles R, R', R" et M représentent un atome d'hydrogène et n est égal à 2. On peut citer en particulier la fécule de pomme de terre modifiée par de l'acide 2-chloroéthyl aminodipropionique neutralisée à la soude, commercialisée sous la référence STRUCTURE SOLANACE par la société NATIONAL STARCH.

De préférence le ou les amidons utilisables dans l'invention sont chimiquement modifiés.

Le ou les polymères différents des polymères associatifs non ioniques décrits précédemment représentent généralement de 0 à 20%, de préférence de 0,2 à 10% en poids, du poids total de la composition.

La composition utilisée selon l'invention peut également comprendre un ou plusieurs agents tensioactifs anioniques, cationiques, amphotères et/ou non ioniques.

Parmi les agents tensioactifs du type anionique pouvant être utilisés dans les compositions utilisées selon l'invention, on peut citer notamment les sels, en particulier les sels alcalins et notamment de sodium, les sels d'ammonium, les sels d'amines, les sels d'aminoal-cools ou les sels de magnésium des composés suivants : les alkylsulfates, les alkyléthersulfates, les alkylamidoéthersulfates, les monoglycérides sulfates, les alkylglycérylsulfonates, les alkylsulfonates, les alkyl-phosphates, les alkylamidesulfonates, les alkylarylsulfonates, les α-oléfinesulfonates, les paraffines sulfonates, les alkylsulfosuccinates, les alkyléthersulfosuccinates, les alkylamidesulfosuccinates, les alkylsulfosuccinamates, les alkylsulfoacétates, les alkylétherphosphates, les acyliséthionates, les N-acyltaurates, les N-acylaminoacides tels que les N-acylsarcosinates et les N-acylglutamates. On peut également citer comme agents tensioactifs anioniques pouvant être utilisés dans les compositions selon l'invention, les sels d'acides gras tels que les sels des acides undécénylique, oléique, ricinoléique, palmitique et stéarique, les acides d'huile de coprah ou d'huile de coprah hydrogénée et les acylhydroxyacides tels que les acyl-lactylates. On peut également utiliser des agents tensioactifs faiblement anioniques tels que les acides d'alkyl D-galactoside uroniques et leurs sels ainsi que les acides alkyléthers alkylamidoéthercarboxyliques polyoxyalkylénés ou leurs sels, le radical alkyle ou acyle de ces différents composés comportant de préférence de 8 à 22 atomes de carbone et les dérivés anioniques d'alkyle (C₈-C₂₂) polyglycosides (sulfate, sulfosuccinate, phosphate, iséthionate, éthercarboxylate, carbonate).

Parmi les agents tensioactifs du type amphotère on peut notamment citer les dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant tel que par exemple, un groupe carboxylate, sulfonate, sulfate, phosphate ou phosphonate. On peut encore citer parmi les agents tensioactifs de type amphotère ou zwittérionique les sulfobétaines, les alkylamidoalkylbétaïnes, les alkylamidoalkylsulfobétaines, les dérivés d'imidazolium tels que ceux d'amphocarboxyglycinate ou d'amphocarboxypropionate.

Parmi les agents tensioactifs de type non ionique utilisables selon l'invention, on peut citer notamment les dérivés polyéthoxylés polypropoxylés ou polyglycérolés des alcools ou des alphadiols ou des alkylphénols ou des acides gras, ayant une chaîne grasse comportant de 8 à 28 atomes de carbone, le nombre de groupes d'oxyde d'éthylène ou de propylène pouvant aller de 2 à 50 et celui de glycérol notamment de 2 à 30. On peut également citer les copolymères d'oxyde d'éthylène et de propylène, les condensats d'oxyde d'éthylène et de propylène sur des alcools gras, les amides gras polyéthoxylés ayant de préférence de 2 à 30 moles d'oxyde d'éthylène, les amides gras polyglycérolés comportant en moyenne 1 à 5 groupes glycérol, les diglycolamides polyglycérolés, les esters d'acides gras du sorbitan éventuellement oxyéthylénés, les esters d'acides gras du saccharose, les esters d'acides gras polyoxyalkylénés, les alkylpolyglycosides éventuellement oxyalkylénés, les esters d'alkylglucosides, les dérivés de N-alkylglucamine et de N-acyl-méthylglucamine, les aldobionamides et les oxydes d'amine.

Comme tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline.

On peut aussi utiliser à titre de tensioactifs cationiques les sels d'ammonium quaternaire contenant au moins une fonction ester tels que ceux de formule (XV) suivante : 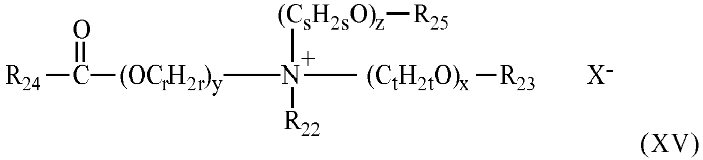 dans laquelle :
R₂₂ est choisi parmi les radicaux alkyles en C₁-C₆ et les radicaux hydroxyalkyles ou dihydroxyalkyles en C₁-C₆ ;
R₂₃ est choisi parmi :
   - le radical 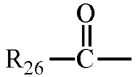
   - les radicaux R₂₇ hydrocarbonés en C₁-C₂₂, linéaires ou ramifiés, saturés ou insaturés,
   - l'atome d'hydrogène,
R₂₅ est choisi parmi :
   - le radical 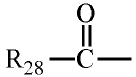
   - les radicaux R₂₉ hydrocarbonés en C₁-C₆, linéaires ou ramifiés, saturés ou insaturés,
   - l'atome d'hydrogène,
R₂₄, R₂₆ et R₂₈, identiques ou différents, sont choisis parmi les radicaux hydrocarbonés en C₇-C₂₁, linéaires ou ramifiés, saturés ou insaturés ;
r, s et t, identiques ou différents, sont des entiers valant de 2 à 6;
y est un entier valant de 1 à 10 ;
x et z, identiques ou différents, sont des entiers valant de 0 à 10 ;
X⁻ est un anion simple ou complexe, organique ou inorganique;
sous réserve que la somme x + y + z vaut de 1 à 15, que lorsque x vaut 0 alors R₂₃ désigne R₂₇ et que lorsque z vaut 0 alors R₂₅ désigne R₂₉.

Les radicaux alkyles R₂₂ peuvent être linéaires ou ramifiés et plus particulièrement linéaires.

De préférence R₂₂ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₂₃ est un radical R₂₇ hydrocarboné, il peut être long et avoir de 12 à 22 atomes de carbone, ou court et avoir de 1 à 3 atomes de carbone.

Lorsque R₂₅ est un radical R₂₉ hydrocarboné, il a de préférence 1 à 3 atomes de carbone.

Avantageusement, R₂₄, R₂₆ et R₂₈, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

Parmi les sels d'ammonium de formule (XV), on utilise plus particulièrement les composés dans lesquels :
- R₂₂ désigne un radical méthyle ou éthyle,
- x et y sont égaux à 1 ;
- z est égal à 0 ou 1 ;
- r, s et t sont égaux à 2 ;
- R₂₃ est choisi parmi :
   - le radical 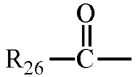
   - les radicaux méthyle, éthyle ou hydrocarbonés en C₁₄-C₂₂,
   - l'atome d'hydrogène ;
- R₂₅ est choisi parmi :
   - le radical 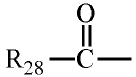
   - l'atome d'hydrogène ;
- R₂₄, R₂₆ et R₂₈, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (XV) tels que les sels (chlorure ou méthylsulfate notamment) de diacyloxyéthyldiméthylammonium, de diacyloxyéthyl-hydroxyéthyl-méthylammonium, de monoacyloxyéthyl-dihydroxyéthylméthylammonium, de triacyloxy éthyl-méthylammonium, de monoacyloxyéthyl-hydroxyéthyl-diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents. On peut citer en particulier le méthosulfate de distéaroyléthyl hydroxyéthylammonium.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthane sulfonate de méthyle, le paratoluènesulfonate de méthyle, la chlorhydrine du glycol ou du glycérol.

La composition utilisée selon l'invention peut contenir un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthyl sulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

A titre d'exemples non limitatifs de tensioactifs convenant pour la réalisation des compositions selon l'invention, on peut citer le REWOPOL SB F 12 P dont l'actif est le laurylsulfosuccinate de sodium, le TEXAPON Z 95 P dont l'actif est le laurylsulfate de sodium, la GENAMIN KDMP dont l'actif est le chlorure de béhényltriméthylammonium, le DEHYQUART F 75 dont l'actif est le méthosulfate de dicétéaroyléthylhydroxyéthylméthylammonium et le TWEEN 21 dont l'actif est le monolaurate de sorbitane à 4 moles d'oxyde d'éthylène.

Lorsqu'ils sont présents, le ou les tensioactifs représentent généralement de 0,1 à 10% en poids du poids total de la composition.

La composition utilisée selon l'invention comprend un milieu cosmétiquement acceptable.

Ce milieu est de préférence aqueux, c'est-à-dire qu'il comprend soit uniquement de l'eau, soit de l'eau et un ou plusieurs solvants tels que par exemple l'éthanol, le propylène glycol, le butylène glycol, l'isopropanol, les éthers de glycol tel que les alkyl (C₁-C₄) éther de mono, di- ou tripropylène glycol, mono, di- ou triéthylène glycol, le dipropylène glycol, le diéthylène glycol et leurs mélanges.

Le milieu peut être également anhydre ou essentiellement anhydre.

La composition utilisée selon l'invention peut en outre comprendre tout additif susceptible d'être utilisé dans le domaine d'application considéré.

Elle est de préférence aqueuse.

En particulier, elle peut comprendre des parfums, des filtres UV, des conservateurs, des antioxydants, des agents régulateurs de pH, des séquestrants, des agents anti-radicaux libres, des hydratants, des agents réducteurs, des agents conditionneurs autres que les silicones, les polymères et les tensioactifs mentionnés précédemment, tels que des esters gras, et des vitamines.

L'invention a encore pour objet une composition cosmétique comprenant dans un milieu cosmétiquement acceptable un ou plusieurs alcanes linéaires volatils et un ou plusieurs polymères associatifs non ioniques, le ou lesdits polymères associatifs non-ioniques étant choisis parmi :
(1) les celluloses modifiées par des groupements comportant au moins une chaîne grasse ;
(2) les hydroxypropylguars modifiés par des groupements comportant au moins une chaîne grasse ;
(3) les copolymères de méthacrylates ou d'acrylates d'alkyles en C₁-C₆ et de monomères amphiphiles comportant au moins une chaîne grasse ;
(4) les copolymères de méthacrylates ou d'acrylates hydrophiles et de monomères hydrophobes comportant comportant au moins une chaîne grasse ;
(5) les polyuréthanes polyéthers comportant dans leur chaîne, à la fois des séquences hydrophiles de nature polyoxyéthylénée et des séquences hydrophobes qui sont des enchaînements aliphatiques seuls et/ou des enchaînements cycloaliphatiques et/ou aromatiques ;
(6) les polymères à squelette aminoplaste éther comportant comportant au moins une chaîne grasse.

Le milieu cosmétiquement acceptable et le ou les alcanes linéaires volatils de la composition selon l'invention peuvent être définis de la même manière que le milieu cosmétiquement acceptable et le ou les alcanes linéaires volatils définis précédemment au sujet de l'utilisation selon l'invention. Le ou les polymères associatifs non ioniques des familles (1) à (6) de la composition selon l'invention peuvent être définis de la même manière que les polymères associatifs non ioniques des familles (1) à (6) définis précédemment au sujet de l'utilisation selon l'invention.

L'invention est illustrée par les exemples qui suivent.

### Exemple 1

On prépare une composition de soin rincé utilisable selon l'invention. La formulation est donnée dans le tableau 1. Les teneurs sont exprimées en g de produit en l'état pour 100g de composition.

**Tableau 1**

| | |
|---|---|
| Mélange myristate/palmitate/stéarate de myristyle/cétyle/stéaryle | 1,5 |
| Poly diméthylsiloxane | 1,5 |
| Homopolymère de chlorure de méthacrylate d'éthyl triméthyl ammonium réticulé en émulsion inverse à 50% dans huile minérale (Salcare SC95 de CIBA) | 1 |
| Distéarate de polyéthylène glycol (150 OE) | 0,3 |
| Cire de candelilla | 2 |
| Phosphate de diamidon de maïs hydroxypropyle prégélatinisé (Structure ZEA) | 0,2 |
| n-Undécane/n-Tridécane selon l'exemple 2 du WO 2008/155059 | 8,5 |
| Alcool cétylstéarylique (C16/C18 50/50) | 8 |
| Copolymère SMDI/Polyéthylène glycol à terminaison alkyle (méthyl/C18) à 15% dans une matrice maltodextrine/eau (Aculyn 46 de ROHM & HAAS) | 3 |
| Eau désionisée | Qsp 100 |
| Conservateurs | qs |
| Parfum | qs |

On applique cette composition sur têtes.

On obtient un apport amélioré de douceur, lissage et souplesse.

### Exemple 2

On prépare une composition de soin rincé utilisable selon l'invention. La formulation est donnée dans le tableau 2. Les teneurs sont exprimées g de produit en l'état pour 100g de composition.

**Tableau 2**

| | |
|---|---|
| Mélange myristate/palmitate/stéarate de myristyle/cétyle/stéaryle (CRODAMOL MS PA de CRODA) | 1,5 |
| Poly diméthylsiloxane (BELSIL DM 300 000 de WACKER) | 1,5 |
| Homopolymère de chlorure de méthacrylate d'éthyl triméthyl ammonium réticulé en émulsion inverse à 50% dans huile minérale (Salcare SC95 de CIBA) | 1 |
| Polyuréthane-39 à 20% de matière active (Luvigel Star de BASF) | 2,2 |
| Distéarate de polyéthylène glycol (150 OE) | 0,3 |
| Cire de candelilla | 2 |
| Phosphate de diamidon de maïs hydroxypropylé prégélatinisé (Structure ZEA de NATIONAL STARCH) | 0,2 |
| n-Undécane/n-Tridécane selon l'exemple 2 du WO 2008/155059 | 8,5 |
| Alcool cétylstéarylique (C₁₆/C₁₈ 50/50) | 8 |
| Eau désionisée | Qsp 100 |
| Conservateurs | qs |
| Parfum | qs |

On applique cette composition sur têtes.

On observe une amélioration des qualités cosmétiques sur cheveux séchés.

### Exemple 3

On prépare une composition de soin non rincé utilisable selon l'invention. La formulation est donnée dans le tableau 4. Les teneurs sont exprimées g de produit en l'état pour 100g de composition.

**Tableau 3**

| | |
|---|---|
| Mélange n-dodécane/n-tétradécane (VEGELIGHT 1214 de BIOSYNTHIS) | 2 |
| Alcool cétylstéarylique (C₁₆/C₁₈ 50/50) | 1 |
| Copolymère SMDI/polyéthylène glycol à terminaison alkyle (méthyl/C₁₈) à 15% dans une matrice maltodextrine/eau (Aculyn 46) | 4 |
| Homopolymère de chlorure de méthacrylate d'éthyl triméthyl ammonium réticulé, en dispersion dans un mélange d'esters à 50% (Salcare SC96) | 1,3 |
| Eau désionisée | Qsp 100 |
| Conservateurs | qs |
| Parfum | qs |

On applique cette composition sur têtes. On observe une amélioration des performances cosmétiques en terme de lissage et de brillance.

### Exemple 4

On prépare une composition de soin non rincé utilisable selon l'invention. La formulation est donnée dans le tableau 4. Les teneurs sont exprimées g de produit en l'état pour 100g de composition.

**Tableau 4**

| | |
|---|---|
| Poly diméthylsiloxane (BELSIL DM 300 000 de WACKER) | 2 |
| n-Dodécane (VEGELIGHT 12 de BIOSYNTHIS) | 2 |
| Copolymère SMDI/polyéthylène glycol à terminaison alkyle (méthyl/C₁₈) à 15% dans une matrice maltodextrine/eau (Aculyn 46 de ROHM 1 HAAS) | 2,5 |
| Homopolymère de chlorure de méthacrylate d'éthyl triméthyl ammonium réticulé, en dispersion dans un mélange d'esters à 50% (Salcare SC96 de CIBA) | 1 |
| Eau désionisée | Qsp 100 |
| Conservateurs | qs |
| Parfum | qs |

On applique cette composition sur têtes.

On observe des performances cosmétiques en terme de douceur, lissage et souplesse améliorées.

### Exemple 5

On prépare une composition de soin non rincé utilisable selon l'invention. La formulation est donnée dans le tableau 5. Les teneurs sont exprimées en g de produit en l'état pour 100g de composition.

**Tableau 5**

| | |
|---|---|
| Poly diméthylsiloxane (BELSIL DM 300 000) | 2 |
| Mélange n-dodécane/n-tétradécane (VEGELIGHT 1214) | 2 |
| Homopolymère de chlorure de méthacrylate d'éthyl triméthyl ammonium réticulé, en dispersion dans un mélange d'esters à 50% (Salcare SC96) | 1 |
| Copolymère SMDI/polyéthylène glycol à terminaison alkyle (méthyl/C18) à 15% dans une matrice maltodextrine/eau (Aculyn 46) | 2,5 |
| Eau désionisée | Qsp 100 |
| Conservateurs | qs |
| Parfum | qs |

On applique cette composition sur têtes.

On observe des performances cosmétiques en terme de douceur, lissage et souplesse améliorées, en particulier sur cheveux séchés.

## Revendications

1. Utilisation pour le conditionnement des fibres kératiniques, en particulier des cheveux, d'une composition cosmétique comprenant dans un milieu cosmétiquement acceptable un ou plusieurs alcanes linéaires comprenant de 7 à 15 atomes de carbone et un ou plusieurs polymères associatifs non ioniques choisis parmi les polyuréthanes polyéthers comportant dans leur chaîne, à la fois des séquences hydrophiles de nature polyoxyéthylénée et des séquences hydrophobes qui sont des enchaînements aliphatiques seuls et/ou des enchaînements cycloaliphatiques et/ou aromatiques.

2. Utilisation selon la revendication précédente **caractérisée en ce que** le ou les alcanes linéaires volatils sont les alcanes linéaires comprenant de 8 à 14 atomes de carbone, mieux de 11 à 14 atomes de carbone.

3. Utilisation selon l'une quelconque des revendications précédentes **caractérisée en ce que** le ou les alcanes linéaires volatils sont d'origine végétale.

4. Utilisation selon l'une quelconque des revendications précédentes **caractérisée en ce que** les alcanes linéaires volatils sont choisis parmi le n- heptane, le n-octane, le n-nonane, le n-décane, le n-undécane, le n-dodécane, le n-tridécane, le n-tétradécane et leurs mélanges.

5. Utilisation selon la revendication 4 **caractérisée en ce que** le ou les alcanes linéaires volatils sont choisis parmi le n-nonane, le n-undécane, le n-dodécane, le n-tridécane, le n-tétradécane et leurs mélanges.

6. Utilisation selon la revendication 5 **caractérisée en ce que** les alcanes linéaires volatils sont un mélange n-undécane/n-tridécane.

7. Utilisation selon l'une quelconque des revendications précédentes **caractérisée en ce que** le ou les alcanes linéaires volatils représentent de 0,5 à 90%, de préférence de 1 à 50% en poids, de préférence encore de 1,5 à 40%, mieux de 2 à 30%, en poids du poids total de la composition.

8. Utilisation selon l'une quelconque des revendications précédentes **caractérisée en ce que** les polyéthers polyuréthanes comportent au moins deux chaînes lipophiles hydrocarbonées, ayant de 8 à 30 atomes de carbone, séparées par une séquence hydrophile, les chaînes hydrocarbonées étant des chaînes pendantes ou des chaînes en bout de séquence hydrophile.

9. Utilisation selon l'une quelconque des revendications précédentes **caractérisée en ce que** le ou les polymères associatifs non ioniques représentent de 0,05 à 10%, de préférence de 0,1 à 5%, et mieux de 0,2 à 2% en poids du poids total de la composition.

10. Utilisation selon l'une quelconque des revendications précédentes **caractérisée en ce que** la composition comprend un ou plusieurs corps gras non siliconés.

11. Utilisation selon l'une quelconque des revendications précédentes **caractérisée en ce que** la composition comprend une ou plusieurs silicones.

12. Composition cosmétique comprenant dans un milieu cosmétiquement acceptable un ou plusieurs alcanes linéaires comprenant de 7 à 15 atomes de carbone et un ou plusieurs polymères associatifs non ioniques, **caractérisée en ce que** le ou les polymères associatifs non-ioniques sont choisis parmi les polyuréthanes polyéthers comportant dans leur chaîne, à la fois des séquences hydrophiles de nature polyoxyéthylénée et des séquences hydrophobes qui sont des enchaînements aliphatiques seuls et/ou des enchaînements cycloaliphatiques et/ou aromatiques.
